# EUROPÄISCHE PATENTSCHRIFT

(11) **EP 3 403 695 B1**
(45) Veröffentlichungstag und Bekanntmachung des Hinweises auf die Patenterteilung: **24.06.2020**
(21) Anmeldenummer: 18171598.8
(22) Anmeldetag: 09.05.2018
(51) Int. Cl.: A61K 8/02, A61K 8/26, A61K 8/37, A61K 8/73, A61K 8/85, A61Q 19/10

(54) **BIOLOGISCH ABBAUBARE REIBEMITTEL**
BIODEGRADABLE ABRASIVE AGENT
AGENT ABRASIF BIODÉGRADABLE

(30) Priorität: 15.05.2017 EP 17171095
(43) Veröffentlichungstag der Anmeldung: 21.11.2018
(73) Patentinhaber: Peter Greven Physioderm GmbH, 53881 Euskirchen (DE)
(72) Erfinder: Stolz, Hermann, 53902 Bad Münstereifel (DE); Nolte, Bert, 53902 Bad Münstereifel (DE); Börnicke, Robert, 53902 Bad Münstereifel (DE)
(74) Vertreter: dompatent von Kreisler Selting Werner - Partnerschaft von Patent- und Rechtsanwälten mbB

(56) Entgegenhaltungen:
- EP-A1- 1 967 173
- EP-A2- 1 106 173
- WO-A1-92/09265
- WO-A1-99/52500
- WO-A1-03/043599

## Beschreibung

Die Erfindung betrifft ein Hautreinigungsmittel, ein Reibemittel für das Hautreinigungsmittel sowie ein Verfahren zur Herstellung des Reibemittels.

Hautreinigungsmittel können zur Verbesserung der Reinigungsleistung Abrasiva enthalten, die die Reinigungswirkung von tensidartigen Komponenten mechanisch unterstützen.

Zahlreiche anorganische und organische Abrasiva sind bekannt, die beispielsweise in Handreinigern oder Peeling-Präparaten eingesetzt werden.

Die zuerst verwendeten Abrasiva waren auf Basis von mineralischen Komponenten wie Quarzsand oder Bimsmehl. Nachteilig hieran ist jedoch, dass Quarzsand und Bimsmehle harte, scharfkantige Körner haben, was zu einer schlechten Hautverträglichkeit führt. Aufgrund ihrer hohen Dichte neigen sie zur Sedimentation und führen damit zu einem Verstopfen der Abflüsse.

Weitere eingesetzte Komponenten sind Holzmehle. Diese haben eine verbesserte Hautverträglichkeit und sedimentieren in den Abflüssen nicht mehr. Allerdings besteht durch die Verwendung von Holzmehl grundsätzlich das Risiko von Allergien gegen Terpeninhaltsstoffe des Holzes. Darüber hinaus ist nachteilig, dass Holzmehl in Verdacht steht, krebserregend zu sein, so dass strenge Sicherheitsvorkehrungen bei der Herstellung und Verarbeitung von Holzmehl zu beachten sind.

Weitere Abrasiva sind auf das Basis von Polyurethan- oder Polyethylenmehl erhältlich. Von ihrer Hautverträglichkeit, der Härte, etc. sind sie hervorragend als Abrasiva geeignet. Allerdings sind sie praktisch biologisch nicht abbaubar, so dass sie in Bezug auf Umweltschutz und Nachhaltigkeit nicht optimal sind.

Die WO 03/043599 A1 beschreibt beispielsweise Hautreinigungszubereitungen, die als Abrasiva beispielsweise Hochdruck Polyethylenpartikel (HD PE) enthalten.

Eine weitere Gruppe von Abrasiva ist auf der Basis von Mehlen von Naturstoffen erhältlich.

Die DE 40 38 076 C2 beschreibt beispielsweise Schalen- und Kernmehle, insbesondere Walnussschalenmehle.

WO 99/52500 beschreibt den Einsatz von Naturmehlen aus gemahlenen Kernen, Schalen oder Samen als Abrasiva.

EP 1 106 173 A2 beschreibt den Einsatz von Maiskolbenmehlen als Abrasiva.

Hierbei ist zu bedenken, dass zum Einsatz eine Desinfektion des eingesetzten Mehles notwendig ist, um eine Verkeimung der Produkte zu vermeiden. Die oben genannte EP 1 106 173 beschreibt ein Bleichverfahren für Naturmehle, das gleichzeitig gegebenenfalls vorhandene Keime abtötet. Während grundsätzlich die Verwendung solcher Naturstoffmehle unter Nachhaltigkeitsgesichtspunkten gut geeignet ist, sind die Eigenschaften solcher Mehle weiterhin nicht optimal.

WO 2008/107453 A2 beschreibt ein Reibemittel auf Basis von hydriertem Rizinusöl. Diese binden aufgrund ihrer Polarität den Schmutz an der Oberfläche der Partikel. Sie lassen sich aber nicht in lösemittelhaltigen Reinigungsprodukten einsetzen. Ihre Reinigungskraft ist nicht so stark wie die von kunststoffbasierten Produkten.

Diese kunststoffbasierten Produkte dominieren aktuell den Markt an Reibemitteln. Größter Nachteil der Produkte ist ihre schlechte biologische Abbaubarkeit. Der Einsatz solcher Produkte wird zunehmend kritisch gesehen.

In der europäischen Kosmetikindustrie wurden im Jahr 2012 circa 4 000 Tonnen solcher Mikrokunststoffe verwendet. Der Eintrag in die Nordsee wird auf 1 bis 1,5 % geschätzt.

In den USA wurde kürzlich der Einsatz von Kunststoffpartikeln in Kosmetika verboten.

Die Anforderungen an Reibemittel sind vielfältig. Sie umfassen folgende Eigenschaften:
- Ökologie: Die biologische Abbaubarkeit des Reibemittels sollte möglichst hoch sein
- Wirksamkeit: Die Reinigungsleistung sollte sich idealerweise an der Reinigungsleistung von aktuellen Polyethylen- oder Polyurethanpartikeln orientieren
- Nachhaltigkeit: Die eingesetzten Rohstoffe sollten möglichst nachhaltig sein, das heißt aus nachwachsenden Quellen stammen
- Geruch: Einige Kern- und Schalenmehle weisen einen deutlichen Eigengeruch auf, der möglichst vermieden werden sollte
- Farbe: Polyethylen ergibt weiße Reibekörper, Polyurethan leicht beigegefärbte Reinigungskörper. Das Produkt sollte idealerweise eine ähnliche Farbe aufweisen
- Stabilität: Das Produkt sollte in wässrigen Zubereitungen stabile Produkte ergeben. Durch Änderung von Schüttgewicht und Dichte lässt sich die Stabilität in einer Zubereitung, aber auch die Sedimentation im Abwasser beeinflussen.
- Hautverträglichkeit: Grundsätzlich stehen sich Reinigungsleistungen und Hautverträglichkeit als Eigenschaften diametral gegenüber. Es muss also für den Reibekörper ein Kompromiss zwischen beiden Parametern gefunden werden.

Es besteht daher Bedarf an Hautreinigungsmitteln, die auf der einen Seite gute Reinigungsleistung bieten und hautschonende Eigenschaften haben, aber biologisch abbaubar sind.

Aufgabe der vorliegenden Erfindung war es, Reibemittel und ein Hautreinigungsmittel enthaltend die Reibemittel bereit zu stellen, die die genannten Nachteile des Standes der Technik zumindest teilweise überwinden, insbesondere eine hohe Umweltverträglichkeit mit sich bringt und möglichst viele der oben genannten Eigenschaften erfüllen.

Gelöst wird die Aufgabe durch ein Hautreinigungsmittel enthaltend
a) 2 bis 25 Gew.-% Reibemittel mit einer mittleren Korngröße von 100 bis 1.000 µm enthaltend
   - mindestens 50 Gew.-% einer Kombination von Celluloseester und Weichmacher
   - 5 bis 45 Gew.-% eines oder mehrere Copolymere aus der Gruppe der Polyhydroxyalkanoate
b) 2 bis 30 Gew.-% Tenside,
c) 0,1 bis 10 Gew.-% Verdickungsmittel,
d) Wasser, sowie gegebenenfalls weitere Hilfsstoffe.

Erfindungsgemäß werden Reibemittel eingesetzt, die Celluloseester enthalten.

Die erfindungsgemäß verwendeten Reibemittel enthalten zumindest 50 Gew.-% einer Kombination von Celluloseester und Weichmacher, bevorzugt liegt die Kombinationen von Celluloseestern und Weichmachern in einer Menge von mindestens 60 Gew.-%, oder mindestens 70 Gew.-%, oder bevorzugt mindestens 80 oder mindestens 90 Gew.-% in den Reibemitteln vor.

Die erfindungsgemäß verwendeten Weichmacher im Reibemittel liegen bevorzugt in einer Menge von mindestens 5 Gew.-% vor. Der Weichmacheranteil im Reibemittel liegt bevorzugt bei nicht mehr als 50 Gew.-%. Bevorzugt werden Weichmacher mit sehr guter biologischer Abbaubarkeit und Lebensmittelzulassung, wie z. B. Triethylcitrat oder Glycerintriacetat verwendet. Weitere geeignete Weichmacher sind Sebacinsäureester, Adipinsäureester, epoxidierte Öle oder Trimellitate, oder andere Glycerin- und Zitronensäureester. Die Verwendung von Glycerin- und Zitronensäureestern ist bevorzugt.

Die erfindungsgemäßen Reibemittel enthalten zusätzlich weitere Inhaltsstoffe, nämlich Copolymere aus der Gruppe der Polyhydroxyalkanoate (PHA). Hierbei handelt es sich um biosynthetisch hergestellte Polyester. Bevorzugte Vertreter sind Poly(3-hydroxybutyrate) (PHB), Poly(3-hydroxybutyrat-co-3-hydroxyvalerate) (PHBV), Poly(3-hydroxybutyrat-co-3-hydroxyhexanoate) (PHBH), insbesondere PHBHₓ.

Diese zeichnen sich durch gute Abbaubarkeit aus.

Beispielhaft liegt ein geeigneter Gehalt an Weichmachern im Reibemittel bei 5 bis 15 Gew.-%, ein Gehalt an Copolymeren aus der Gruppe der Polyhydroxyalkanoate zwischen 5 und 20 Gew.-% und an Celluloseester zwischen 60 bis 90 Gew.-%, wobei dies zusammen mit gegebenenfalls vorhandenen weiteren Inhaltsstoffen 100 Gew.-% ergibt.

Der Gehalt an Reibemittel im Hautreinigungsmittel liegt bevorzugt bei mindestens 3 Gew.-%, mehr bevorzugt bei mindestens 5 Gew.-%, noch mehr bevorzugt bei mindestens 6 Gew.-% oder mindestens 7 Gew.-%.

Als Tenside zur Verwendung im Hautreinigungsmittel haben sich insbesondere nicht-ionische, zwitter-ionische und anionische Tenside als geeignet erwiesen, beispielsweise Ethersulfate, Betaine, Alkylsulfonate, Succinate, Alkypolygylcoside, Eiweißfettsäurekondensate, Polyglycolether, Seifen und Mischungen davon. Die Auswahl entsprechender Tenside ist dem Fachmann geläufig.

Als Verdickungsmittel eignen sich insbesondere Bentonite, Xanthane, Acrylate, Alginate, Celluloseether, Carrageen und Mischungen davon.

Zusätzlich kann das erfindungsgemäße Hautreinigungsmittel einen oder mehrere der folgenden üblichen Zusatzstoffe enthalten:
- Rückfetter,
- Farbstoffe,
- Parfümstoffe,
- Titandioxid,
- Puffersubstanzen,
- Konservierungsmittel,
- flüssige Parafine,
- Glycerin,
- Antioxidationsmitteln,
- weitere Abrasiva
- Lösungsmittel.

Durch den Gehalt an Verdickungsmitteln und den Gehalt an Reibemitteln lässt sich die Fließfähigkeit des Hautreinigungsmittels einstellen. Mit geringen Gehalten an Verdickungsmitteln und Reibemitteln werden Fließpasten erhalten. Mit relativ hohen Gehalten werden feste Reinigungspasten erhalten, wie sie beispielsweise zur Handreinigung häufig eingesetzt werden.

Bevorzugt liegt das Produkt als wässrige Suspension vor. Eine Öl-in-Wasser Emulsion ist weniger bevorzugt.

Die Reinigungswirkung der Reibemittel kann durch die Korngröße und das Herstellverfahren beeinflusst werden. Grundsätzlich geben größere Reibemittel eine stärkere Reinigungswirkung.

Gegenstand der Erfindung sind auch Reibemittel für kosmetische Präparate mit einer mittleren Korngröße von 100 bis 1.000 µm, die zu mindestens 50 Gew.-% einer Kombination von Celluloseestern und Weichmachern und zu 5 Gew.-% bis 45 Gew.-% ein Copolymer aus der Gruppe der Polyhydroxyalkanoate enthalten.

Hergestellt wird der erfindungsgemäße Reinigungskörper bevorzugt durch ein Verfahren zur Herstellung des Reibemittels umfassend die Schritte
- Verschäumen einer Mischung enthaltend
- mindestens 50 Gew.-% einer Kombination von Celluloseester und Weichmacher im Extruder
- 5 Gew.-% bis 45 Gew.-% eines Copolymers aus der Gruppe der Polyhydroxyalkanoate
- Vermahlen des enthaltenen Schaums.

Ein weiterer Gegenstand der Erfindung ist die Verwendung der erfindungsgemäßen Reibemittel als Inhaltsstoffe von Kosmetika, insbesondere Hautreinigungsmitteln.

Aspekte sind auch:
(1) Hautreinigungsmittel enthaltend
   a) 2 bis 25 Gew.-% Reibemittel mit einer mittleren Korngröße von 100 bis 1.000 µm enthaltend
      - mindestens 50 Gew.-% einer Kombination von Celluloseester und Weichmacher
      - 5 Gew.-% bis 45 Gew.-% eines Copolymers aus der Gruppe der Polyhydroxyalkanoate
   b) 2 bis 30 Gew.-% Tenside,
   c) 0,1 bis 10 Gew.-% Verdickungsmittel,
   d) Wasser, sowie gegebenenfalls weitere Hilfsstoffe.
(2) Hautreinigungsmittel nach (1), wobei der Celluloseester mindestens 5 Gew.-% Weichmacher enthält. Bevorzugt werden Weichmacher, mit sehr guter biologischer Abbaubarkeit und Lebensmittelzulassung, wie z. B. Zitronensäureester.
(3) Hautreinigungsmittel nach (1) oder (2), wobei das Reibemittel zwischen 0 und 5 Gew.-% Füllstoffe, wie Talkum, Kaolin, Kreide oder Kieselsäuren enthält.
(4) Hautreinigungsmittel nach (1) bis (3), wobei das Verdickungsmittel aus Bentoniten, Xanthanen, Acrylaten, Alginaten, Cellulose-Ethern, Carrageen und Mischungen davon ausgewählt wird.
(5) Hautreinigungsmittel nach (1) bis (4), wobei als Hilfsstoffe zusätzlich eine oder mehrere der folgenden Substanzen enthalten sind
   - Rückfetter,
   - Farbstoffe,
   - Parfümstoffe,
   - Titandioxid,
   - Puffersubstanzen
   - Konservierungsmittel
   - flüssige Paraffine
   - Glycerin,
   - Antioxidationsmitteln,
   - Abrasiva
   - Lösungsmittel.
(6) Reibemittel für kosmetische Präparate, insbesondere Hautreinigungsmittel, mit einer mittleren Korngröße von 100 bis 1.000 µm, die
   - mindestens 50 Gew.-% einer Kombination von Celluloseestern und Weichmachern
   - 5 Gew.-% bis 45 Gew.-% eines Copolymers aus der Gruppe der Polyhydroxyalkanoate enthalten.
(7) Reibemittel nach (6), enthaltend mindestens 5 Gew.-% eines Weichmachers.
(8) Reibemittel nach (6) und (7), wobei das Copolymer aus der Gruppe der Polyhydroxyalkanoate bevorzugt PHB, PHBV, PHBH, insbesondere PHBHₓ ist.
(9) Verfahren zur Herstellung des Reibemittels nach (6) bis (8) umfassend die Schritte
   - Verschäumen einer Mischung enthaltend mindestens 50 Gew.-% Celluloseester und Weichmacher im Extruder
   - 5 Gew.-% bis 45 Gew.-% eines Copolymers aus der Gruppe der Polyhydroxyalkanoate
   - Vermahlen des enthaltenen Schaums.
(10) Verwendung von Reibemitteln nach mindestens (6) bis (8) als Inhaltsstoffe von Kosmetika, insbesondere Hautreinigungsmitteln.

### Beispiel 1: Screening von Biokunststoffen

Es wurden verschiedene sogenannte Biokunststoffe eingesetzt. Dabei handelt es sich um Polylactide (PLA), Polyhydroxyalkanoate (PHA), Stärkederivate (natürliche Stärke, Stärke 9030, eine Glycosylstärke erhältlich von AGRANA, Österreich, Stärke 8935, eine thermoplastische Stärke von AGRANA, Österreich), Celluloseacetat (CA), Polybutylensuccinat (PBST) und Polycaptrolacton (PCL). Die entsprechenden Kunststoffe wurden compoundiert, mit Hilfe eines chemischen Treibmittels (Natriumhydrogencarbonat/Zitronensäure) verschäumt und anschließend vermahlen. Damit wurden folgende Materialien hergestellt:

| **Produkt** | **Dichte kg/m³** | **D50 µm** |
|---|---|---|
| | **(Pyknometer)** | |
| PLA | 592,7 | 487 |
| Blend 1: PLA/nat. Stärke | 558,6 | 370 |
| Blend 2: PLA/Stärke 9030 | 626,2 | 469 |
| Blend 3: PLA/Stärke 8935 | 745,9 | 321 |
| Blend 4: PLA/PBST/PLA | 589,1 | 460 |
| Blend 5: PLA/PCL | 603,5 | 466 |
| Weichgemachtes CA | 208,9 | 227 |

Anschließend wurden die Produkte einem Test ihrer Abbauraten unterzogen. Der Test funktionierte wie folgt:
Die Abbaubarkeit wurde gemessen durch den Sauerstoffverbrauch in einem geschlossenen Respiro-Meter. Als Inokulum diente eine Probe aus der Kläranlage Breisgauer Bucht. Die Bestimmung erfolgte nach ISO 14851 aus dem Verhältnis von aktuellem zum theoretischen Sauerstoffverbrauch.

Es zeigte sich, dass viele der eingesetzten Produkte schlechte Abbaubarkeiten aufwiesen:

| **Produkt** | **Abbaubarkeit** |
|---|---|
| | **in % von ThOD (Theoretical oxygen demand)** |
| Cellulose | 92 |
| PLA | 0 |
| Blend 1: PLA/nat. Stärke | 8 - 10 |
| Blend 2: PLA/Stärke 9030 | 5 - 6 |
| Blend 3: PLA/Stärke 8935 | 6 - 9 |
| Blend 4: PLA/PBST/PLA | 2 - 3 |
| Blend 5: PLA/PCL | 7 - 10 |
| Weichqemachtes CA | 20 - 25 |
| Karnaubawachs | 0 |

Von den getesteten Produkten zeigt nur das weichgemachte Celluloseacetat eine gute Abbaubarkeit.

Es wurden folgende weitere Produkte hergestellt. Dazu wurde Celluloseacetat oder Ethylcellulose mit Polyhydroxybuttersäurecovalerat (PHBV) oder Triethylcitrat (TEC) eingesetzt. Es wurden die folgenden Produkte hergestellt und die Schmelzflussrate (MFI) bestimmt:

| Produkt | MFI | Dichte | D50 | ThOD |
|---|---|---|---|---|
| | g/ 10 min (bei 230 °C) | g/ml | µm | % |
| CA mit 15% TEC | 6,9 | 107,5 | 91,3 | 26 |
| CA mit 7,5% PHBV + 10% TEC | 4,5 | 102,6 | 76,9 | 34 |
| CA mit 15% PHBV + 10% TEC | 13,1 | 203,9 | 82,9 | 40 |
| CA mit 15% hydrophobierter thermoplastischer Stärke* + 10% TEC | 3,7 | 107,5 | 89,1 | 30 |
| CA mit 15%EC + 10% TEC | 13,8 | keine Schaumbildung | 102,1 | N.A. |
| EC mit 15% PHBV | keine Verarb. möglich | | | N.A. |
| CA-Schaum physikalisch qeschäumt | n. comp | 115,2 | 139,4 | 31 |
| Olivenkernmehl | ---- | ---- | ---- | 30 |
| Walnußschalenmehl | ---- | ---- | ---- | 9 |

| | | | | |
|---|---|---|---|---|
| *Produkt Agenaflo 9050 der Firma Agrana | | | | |

Mit Ausnahme des Produktes Ethylcellulose mit PHBV ließen sich alle Produkte einwandfrei compoundieren. Es gab allerdings Probleme bei der Schaumbildung. Alle Produkte zeigten ein niedrigen Melt-Flow-Index (MFI), der eine spätere Verschäumung erleichtert. Als Treibmittel wurden Zitronensäure und Natriumhydrogencarbonat verwendet. Alle Produkte ergaben einen stabilen Schaum der stufenweise in einer Siebmühle gemahlen wurde.

Nach 56 Tagen zeigte sich eine Abbaubarkeit, die deutlich über der von Wallnussschalen lag, siehe Tabelle. Olivenkernmehle sind trotz gröberer Fraktion besser abbaubar als Wallnussschalenmehle. Es zeigt sich auch, dass die Zugabe von PHBV die Abbaurate des Celluloseesters deutlich erhöht. Die Abbaubarkeit der entsprechenden Produkte liegt deutlich über der von Wallnussschalmehl und auf vergleichbarem Niveau wie Olivenkernmehl.

Die Cellulose stammt zu 100% aus nachwachsenden Rohstoffen. Auch das Essigsäureanhydrid kann aus biogenen Quellen stammen, sodass die Produkte nachhaltig sind.

In einem nächsten Schritt wurde Celluloseacetat (physikalisch geschäumt) im Vergleich zu Celluloseacetat mit Triethylacetat und PHBV getestet. Es ergaben sich folgende physikalische Werte:

| **Schaum** | **Druckmodul** | **Druckfestigkeit 10% Stauchung** | **Dichte** | **Schüttdichte (250µ)** |
|---|---|---|---|---|
| | kPa | kPa | kg/m³ | g/l |
| Polyurethan (Referenz) | 2522 | 195 | 35,67 | 141,0 |
| CA/10% TEC/15% PHBV | 23149 | 1542 | 203,94 | 180,5 |
| CA-Schaum physikalisch geschäumt | 21492 | 733 | 115,20 | 275,3 |

Beide Produkte hatten eine weitgehend weiße Farbe und keinen signifikanten Eigengeruch.

### Beispiel 2:

Es werden folgende Zusammensetzungen hergestellt:

| **Bezeichnung** | **Anteil (%)** |
|---|---|
| SODIUM COCO SULFATE | 13,00 |
| DISODIUM LAURETH SULFOSUCCINATE | 7,00 |
| ALLANTOIN | 0,40 |
| SODIUM BENZOATE | 0,30 |
| POTASSIUM SORBATE | 0,15 |
| LACTIC ACID | 0,25 |
| XANTHAN GUM | 0,50 |
| PARFUME | 0,20 |
| GLYCERINE | 4,00 |
| Reibemittel CA/TEC/PHBV [75/10/15] | 16,00 |
| BRASSICA CAMPESTRIS | 16,20 |
| AQUA | 42,00 |

Bei der Rezeptur handelt es sich um einen pastösen Hautreiniger aus Basis von Cocosulfat und Laureth-Sulfosuccinat. Das Produkt wird im industriellen Bereich zur Entfernung von hartnäckigen Hautverschmutzungen wie Schmierfette, Ruß und Schmierölen verwendet. Unterstützt werden die Lösungseigenschaften durch Rapsöl. Entsprechend der Anwendung wird ein eher hoher Anteil von 16 % Reibemittel verwendet.

### Beispiel 3:

| **Bezeichnung** | **Anteil (%)** |
|---|---|
| AQUA | ad 100 |
| PARFUM | 0,14 |
| TITANIUM DIOXIDE | 0,30 |
| Reibemittel CA/TEC/PHBV [75/10/15] | 5,00 |
| XANTHAN GUM | 0,50 |
| KAHLGUM TQS 80 | 0,20 |
| SODIUM LAURETH SULFATE | 10,01 |
| COCAMIDOPROPYL BETAIN | 4,20 |
| OLEIC ACID | 0,31 |
| BENTONITE | 2,20 |
| 2-BROMO-2-NITROPROPANE-1,3-DIOL | 0,90 |
| CITRIC ACID | 0,21 |

In der Rezeptur sind die Bestandteile eines pastösen Reinigers gegen mittlere Verschmutzungen der Haut wiedergegeben. Die Tensidbasis bildet Ethersulfat und Betain. Als Konsistenzgeber wird eine Kombination aus Xanthan Gum und Bentonit verwendet. Trotz der niedrigen Dosierung des Reibemittels von 5 % wird eine gute Reinigungsleistung, auch bei haftenden Verschmutzungen, erreicht

Sowohl Beispiel 2 als auch Beispiel 3 ergaben Produkte, die im Vergleich zu entsprechenden Produkten, die Reibemittel aus Polyurethan enthielten, vergleichbare Reinigungseigenschaften aufwiesen.

## Patentansprüche

1. Hautreinigungsmittel enthaltend
a) 2 bis 25 Gew.-% Reibemittel mit einer mittleren Korngröße von 100 bis 1.000 µm enthaltend
- mindestens 50 Gew.-% einer Kombination von Celluloseester und Weichmacher
- 5 bis 45 Gew.-% eines Copolymers aus der Gruppe der Polyhydroxyalkanoate
b) 2 bis 30 Gew.-% Tenside,
c) 0,1 bis 10 Gew.-% Verdickungsmittel,
d) Wasser, sowie gegebenenfalls weitere Hilfsstoffe.

2. Hautreinigungsmittel nach Anspruch 1, wobei das Reibemittel mindestens 5 Gew.-% Weichmacher enthält.

3. Hautreinigungsmittel nach einem der Ansprüche 1 oder 2, wobei das Reibemittel zwischen 0 und 5 Gew.-% Füllstoffe, wie Talkum, Kaolin, Kreide oder Kieselsäuren enthält.

4. Hautreinigungsmittel nach einem der Ansprüche 1 bis 3, wobei das Verdickungsmittel aus Bentoniten, Xanthanen, Acrylaten, Alginaten, Cellulose-Ethern, Carrageen und Mischungen davon ausgewählt wird.

5. Hautreinigungsmittel nach einem der Ansprüche 1 bis 4, wobei als Hilfsstoffe zusätzlich eine oder mehrere der folgenden Substanzen enthalten sind
- Rückfetter,
- Farbstoffe,
- Parfümstoffe,
- Titandioxid,
- Puffersubstanzen
- Konservierungsmittel
- flüssige Paraffine
- Glycerin,
- Antioxidationsmitteln,
- Abrasiva
- Lösungsmittel.

6. Reibemittel für kosmetische Präparate, insbesondere Hautreinigungsmittel, mit einer mittleren Korngröße von 100 bis 1.000 µm, die
- mindestens 50 Gew.-% einer Kombination von Celluloseestern und Weichmachern
- 5 Gew.-% bis 45 Gew.-% eines Copolymers aus der Gruppe der Polyhydroxyalkanoate
enthalten.

7. Reibemittel nach Anspruch 6, enthaltend mindestens 5 Gew.-% eines Weichmachers.

8. Reibemittel nach den Ansprüchen 6 und 7, wobei das Copolymer aus der Gruppe der Polyhydroxyalkanoate PHB, PHBV, PHBH, insbesondere PHBHₓ ist.

9. Verfahren zur Herstellung des Reibemittels nach einem der Ansprüche 6 bis 8 umfassend die Schritte
- Verschäumen einer Mischung enthaltend
- mindestens 50 Gew.-% einer Kombination von Celluloseester und Weichmacher im Extruder
- 5 Gew.-% bis 45 Gew.-% eines Copolymers aus der Gruppe der Polyhydroxyalkanoate
- Vermahlen des enthaltenen Schaums.

10. Verwendung von Reibemitteln nach mindestens einem der Ansprüche 6 bis 8 als Inhaltsstoffe von Kosmetika, insbesondere Hautreinigungsmitteln.

## Claims

1. A skin cleaning agent comprising
a) from 2 to 25% by weight of an abrasive having a mean grain size of from 100 to 1000 µm, comprising
- at least 50% of a combination of cellulose ester and plasticizer;
- from 5 to 45% by weight of a copolymer from the group of polyhydroxyalkanoates;
b) from 2 to 30% by weight of surfactants;
c) from 0.1 to 10% by weight of thickeners;
d) water and optionally further auxiliaries.

2. The skin cleaning agent according to claim 1, wherein said abrasive comprises at least 5% by weight of plasticizers.

3. The skin cleaning agent according to either of claims 1 or 2, wherein said abrasive comprises from 0 to 5% by weight of fillers, such as talcum, china clay, chalk, or silicic acids.

4. The skin cleaning agent according to any of claims 1 to 3, wherein said thickener is selected from bentonites, xanthan gums, acrylates, alginates, cellulose ethers, carrageenan, and mixtures thereof.

5. The skin cleaning agent according to any of claims 1 to 4, wherein one or more of the following substances are additionally contained as said auxiliaries:
- refatting agents;
- colorants;
- perfumes;
- titanium dioxide;
- buffer substances;
- preservatives;
- liquid paraffins;
- glycerol;
- antioxidants;
- abrasives;
- solvents.

6. Abrasives for cosmetic preparations, especially skin cleaning agents, having a mean grain size of from 100 to 1000 µm, comprising
- at least 50% of a combination of cellulose ester and plasticizer;
- from 5 to 45% by weight of a copolymer from the group of polyhydroxyalkanoates.

7. The abrasives according to claim 6, comprising at least 5% by weight of a plasticizer.

8. The abrasives according to claims 6 and 7, wherein said copolymer is selected from the group of polyhydroxyalkanoates PHB, PHBV, PHBH, especially PHBHₓ.

9. A process for preparing the abrasive according to any of claims 6 to 8, comprising the steps of:
- foaming a mixture comprising
- at least 50% of a combination of cellulose ester and plasticizer in an extruder;
- from 5% by weight to 45% by weight of a copolymer selected from the group of polyhydroxyalkanoates.
- grinding the foam obtained.

10. Use of abrasives according to at least one of claims 6 to 8 as ingredients of cosmetics, especially skin cleaning agents.

## Revendications

1. Produit de nettoyage de la peau contenant
a) 2 à 25 % en poids de microbilles avec une granulométrie moyenne de 100 à 1 000 µm contenant
- au moins 50 % en poids d'une combinaison d'ester de cellulose et de plastifiant
- 5 à 45 % en poids d'un copolymère issu du groupe des poly(hydroxyalcanoates)
b) 2 à 30 % en poids de tensioactifs,
c) 0,1 à 10 % en poids d'agents épaississants ;
d) de l'eau, ainsi éventuellement que des adjuvants ultérieurs.

2. Produit de nettoyage de la peau selon la revendication 1, dans lequel les microbilles contiennent au moins 5 % en poids de plastifiant.

3. Produit de nettoyage de la peau selon l'une des revendications 1 ou 2, dans lequel les microbilles contiennent entre 0 et 5 % en poids de charges, telles que du talc, du kaolin, de la craie ou de la silice.

4. Produit de nettoyage de la peau selon l'une des revendications 1 à 3, dans lequel l'agent épaississant est sélectionné parmi les bentonites, les xanthanes, les acrylates, les alginates, les éthers de cellulose, les carraghénanes et les mélanges de ceux-ci.

5. Produit de nettoyage de la peau selon l'une des revendications 1 à 4, dans lequel une ou plusieurs des substances suivantes sont en outre contenues en tant qu'adjuvants
- agent regraissant,
- colorants,
- parfums,
- dioxyde de titane,
- substances tampons
- agent de conservation
- paraffine liquide
- glycérol,
- agents antioxydants,
- abrasifs
- solvant.

6. Microbilles pour préparations cosmétiques, en particulier produits de nettoyage de la peau, avec une granulométrie moyenne de 100 à 1 000 µm, contenant
- au moins 50 % en poids d'une combinaison d'esters de cellulose et de plastifiants
- 5 % en poids à 45 % en poids d'un copolymère issu du groupe des poly(hydroxyalcanoates).

7. Microbilles selon la revendication 6 contenant au moins 5 % en poids de plastifiant.

8. Microbilles selon les revendications 6 et 7, dans lesquelles le copolymère est issu du groupe des poly(hydroxyalcanoates) PHB, PHBV, PHBH, en particulier PHBHₓ .

9. Procédé de production des microbilles selon l'une des revendications 6 à 8, comprenant les étapes :
- moussage d'un mélange contenant
o au moins 50 % en poids d'une combinaison d'ester de cellulose et de plastifiant dans l'extrudeuse
o 5 % en poids à 45 % en poids d'un copolymère issu du groupe des poly(hydroxyalcanoates)
- Broyage de la mousse contenue.

10. Utilisation de microbilles selon au moins une des revendications 6 à 8 en tant qu'ingrédients de cosmétiques, en particulier de produits de nettoyage de la peau.
